# EUROPEAN PATENT APPLICATION

(11) **EP 1 752 115 A1**
(43) Date of publication of application: **14.02.2007**
(21) Application number: 05017220.4
(22) Date of filing: 08.08.2005
(51) Int. Cl.: A61F 2/08

(54) **Surgical device for connecting two anatomical structures**

(71) Applicant: Maselli, Daniele, 56012 Fornacette PI (IT)
(72) Inventor: Maselli, Daniele, 56012 Fornacette PI (IT)
(74) Representative: Celestino, Marco

(57) **Abstract**

A surgical device for connecting two anatomical structures, such as a prosthesis for tendons, ligaments and tendinous cords, or a pulling suture element, comprising a first elongated flexible element (10) having a plurality of enlargements (11), for example knots, and a second elongated flexible element (20) having at one end a slipknot or loop (21). In particular, an end (12) of the first elongated flexible element (10) is constrained to a first anatomical structure (71) and an end (22) of the second flexible elongated element (20), is constrained to a second anatomical structure (72) connected to the first by the damaged ligament or tendon that is intended to be replaced with the prosthesis. Once connected the ends (12, 22) to the respective anatomical structures the elongated flexible elements (10, 20) are arranged according to a positioning configuration that allows a relative movement. More in detail, the relative location of the elongated flexible elements (10, 20) changes by causing the loop (21) to slide along the first elongated flexible element (10) up to a determined point. Then the loop (21) is tightened on the elongated flexible element (10) obtaining a first tightened configuration. During the surgical operation the prosthesis or the suture is applied and its length is checked as corresponding to that necessary to provide a correct relative movement between the two anatomical structures (71, 72) from it connected. In case of incorrect length the loop (21) is loosened and caused to slide along the first element (10) up to the next or previous knot (11) in order achieve a desired functional length.

## Description

### Field of the invention

The present invention generally relates to devices used in a medical field for repairing surgery operations and, in particular, it relates to a surgical device for connecting two anatomical structures, such as, in particular, a prosthesis, consisting of tendons or ligaments that should replace damaged tendons or ligaments, or a suture, suitable for keeping the two structures at a predetermined distance from each other.

### Description of the prior art

As well known, a variety exists of types of prostheses for ligaments and tendons that are used during delicate surgery operations for replacing corresponding damaged anatomical parts.

A repairing surgery operation that is particularly critical relates to the change of the tendinous cords of the cardiovascular system. In the heart the oxygenated blood coming from the lungs is collected in the left atrium and, from here, through the mitral valve, it passes through the left ventricle, which pumps then the blood to all the body. The main function of the mitral valve is to avoid that the blood, during the ventricular contraction, is pumped back to the left atrium. The two leaflets of the mitral valve are held by a system of ties, i.e. the tendinous cords, fixed to the muscular "columns", the papillary muscles, which control their tension. A coordinate contraction of the papillary muscles causes a progressive increase of the tension of the tendinous cords and the adhesion of the opposite valve leaflets, preventing them from turning towards the atrial cavity and assuring the continence of the valve. Most dysfunctions of the mitral valve are associated to an excessive weakness of the structure of the leaflets or of the tendinous cords that can strain or even break them.

In order to surgically restore a correct relationship between the components of the valve, a possible treatment is to shorten the tendinous cords, replacing them with artificial filaments of biocompatible material, for example Gore-Tex®. To carry out effectively their function of ties, and to follow the natural movement of the mitral leaflets during the diastolic and systolic phases, the resilient filaments must have a very precise length. Therefore, during the operation the surgeon must analyse the functionality of the artificial filaments to identify their correct length. For this purpose, a filament is connected at one end to the walls of a mitral leaflet and at the other end to the papillary muscles. However, not always the surgeon succeeds at a first instance to choose the correct length for the filament, and an incorrect length can affect the soundness of the anatomical structures involved in the operation. If during the operation the length chosen is not suitable to ensure the correct functionality of the anatomical structures to be connected, it is necessary that the surgeon cuts the filament and replaces it with a new one, with a subsequent suffering for the anatomical structures.

Other treatments are possible where tendons, ligaments or tendinous cords are added or replaced. For example, common treatments of changing damaged ligaments and tendons involve the knee and the elbow. in even further treatments, variable length sutures are used by surgeons to cause certain parts to have a predetermined length in order to recover predetermined shapes. For example, for correcting strabismus it is necessary to execute a pulling suture having a very precise length for causing muscular tissues of the eye to achieve a predetermined shortening. Even in this case, a suture that has an incorrect length should be removed and repeated, with subsequent suffering caused to the anatomical structures.

### Summary of the invention

It is then a feature of the present invention to provide a variable length surgical device for connecting two anatomical structures without any need to change the connection to the structures during an operation.

It is a particular feature of the present invention to provide a prosthesis for tendons, ligaments and tendinous cords, which allows speeding up and making easier a repairing surgery operation.

It is another particular feature of the present invention to provide a suture suitable for connecting at a predetermined distance two anatomical structures and that is easy and effective for a surgeon.

It is still another particular feature of the present invention to provide a suture or a prosthesis for tendons, ligaments and tendinous cords, which is easily implemented in an implantation site, in particular with a desired length.

It is still another particular feature of the present invention to provide a prosthesis for replacing damaged tendinous cords of a cardiac valve, in species the mitral valve.

These and other objects are achieved by the surgical device for connecting two anatomical structures of a subject, in particular, a prosthesis for tendons, ligaments and tendinous cords, or a pulling suture element, according to the invention, comprising:
- a first elongated flexible element suitable for being constrained at one end to the first anatomical structure;
- a second elongated flexible element suitable for being constrained at one end to the second anatomical structure, said second anatomical structure forming with the first anatomical structure an articular junction;
whose main feature is that said first and said second elongated flexible element have adjustable tightening means providing a mutual engagement to achieve a measured length of the device.

In particular, the adjustable tightening means can be shifted from a positioning configuration, which allows a relative movement of the flexible elongated elements, to a tightened configuration in which the two flexible elongated elements are integral to each other.

In particular, the adjustable tightening means can provide:
- a slipknot or loop executed at the free end of the second elongated flexible element;
- a plurality of enlargements distributed at least on one portion of the first elongated flexible element, said enlargements being suitable for defining determined points on the first elongated flexible element at which said slipknot or loop can be fixed to provide a desired resulting length.

Advantageously, the enlargements are sized such that they block the run of the tightened loop along the first elongated flexible element. In a particular exemplary embodiment of the invention, the enlargements are arranged according to equidistant points distributed on the above described portion of the first elongated flexible element.

In particular, the enlargements can be one or more knots previously prepared. Alternatively, the enlargements can be directly integrated in the body same of the material of the flexible element. In a further alternative, the enlargements can provide elements constrained to the corresponding elongated flexible element, in a fixed way or a longitudinally adjustable way; or they can provide a combination of the above.

The elongated flexible elements can be constrained to the respective anatomical structures by means of fastening means, for example knots, or hooks of biocompatible fastening material.

Advantageously, each elongated flexible element has at one end at least one needle suitable to assist its introduction in the anatomical structure.

Preferably, the elongated flexible elements are made of a material selected from the group comprised of: polyethylenterephtalate (Dacron®), polytetrafluorethylene (Gore-Tex®), polyaramid fibres (Kevlar®) , tetrapoliethylene.

According to a particular aspect of the invention, a prosthesis for tendons, ligaments and tendinous cords for repairing treatment of the mitral valve, as above described, provides that:
- The first elongated flexible element is constrained to a papillary muscle;
- The second elongated flexible element is constrained near a mitral valve leaflet of the corresponding ventricle.

Such exemplary embodiment is used for replacing a damaged tendinous cord of a mitral valve or in any case a tendinous cord that is not more capable of properly controlling the position of the valve leaflets, for example because affected by known pathologies of the mitral valve.

### Brief description of the drawings

The invention will be made clearer with the following description of an exemplary embodiment thereof, exemplifying but not limitative, with reference to the attached drawings wherein:
- figure 1 shows a perspective elevational side view of a first elongated flexible element of a surgical device according to the present invention, in particular a prosthesis of tendinous cord, or a suture;
- figure 2 shows a perspective elevational side view of a second elongated flexible element that forms, with the first elongated flexible element of figure 1, the surgical device according to the present invention;
- figures from 3 to 5 show perspective side elevational views of three possible configurations of the flexible elongated elements of figures 1 and 2;
- figure 6 shows a perspective elevational side view in operative conditions of the prosthesis for tendons, ligaments and tendinous cords according to the invention;
- figure 7 shows diagrammatically a cross section of a human heart explaining the application of an exemplary embodiment of the prosthesis for tendons, ligaments and tendinous cords of figures 1 and 2 during a surgical operation;
- figure 8 shows diagrammatically an enlarged view of a portion of the left ventricle of the heart of figure 2 showing the shape in use of the tendinous cords;
- figure 9 shows diagrammatically the relative position of the tendinous cords with respect to the papillary muscles and to the ventricular mitral leaflets of the heart of figure 6;
- figures 10 and 11 show diagrammatically perspective elevational side views of the prosthesis for tendons or ligaments, used for replacing a damaged tendinous cord of a mitral valve in operative conditions;
- figures 12 and 13 show transversal cross sections of an exemplary embodiment of the enlargements provided on the first elongated flexible element shown in figures 1, 3, 5 and 6;
- figure 14 shows a perspective elevational side view of an exemplary embodiment of an elongated flexible element alternative to that of figure 1;
- figure 15 shows diagrammatically a top plan view of an alternative exemplary embodiment of the elongated flexible element of figure 2;
- figure 16 shows a perspective elevational side view of a step of arranging the flexible element of figure 1 with respect to the elongated element of figure 15;
- figure 17 shows a top plan view of a further exemplary embodiment of the elongated element of figure 1;
- figure 18 shows in an elevational side view of a further exemplary embodiment of the element of figure 1.

### Description of preferred exemplary embodiments.

Notwithstanding in the following description reference is made to a surgical device for connection of two anatomical structures in the form of a prosthesis for tendinous cords, relatively to the mitral valve, it is apparent that the invention can be used in general as prosthesis for tendons, ligaments and tendinous cords or as pulling suture having a predetermined length.

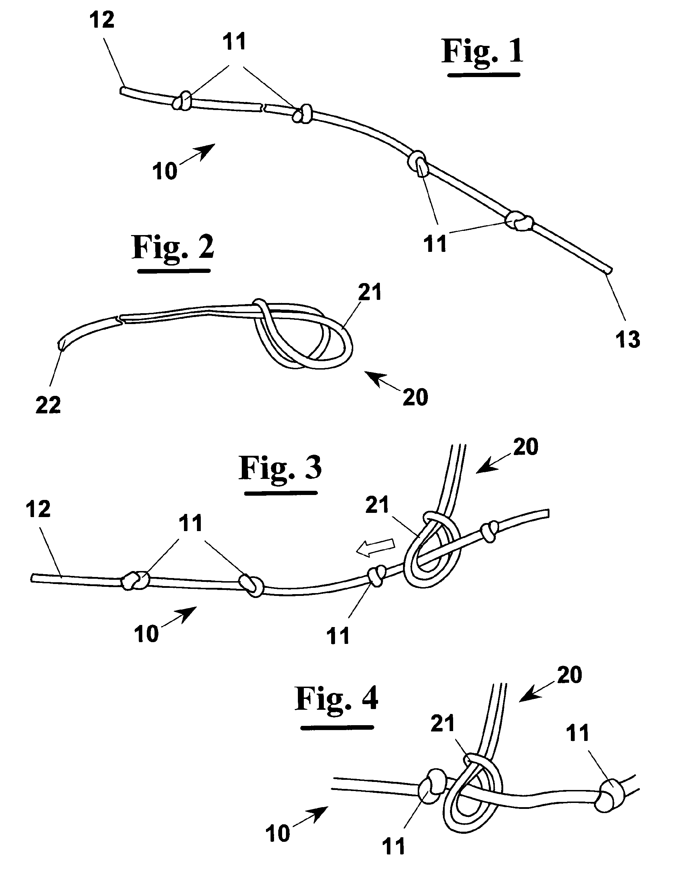

With reference to figures 1 and 2, a prosthesis for tendons, ligaments and tendinous cords according to the present invention provides a first elongated flexible element 10 having a plurality of enlargements 11, for example knots, and a second elongated flexible element 20 having at one end a slipknot or loop 21. In particular, an end 12 of the first elongated flexible element 10 is constrained to a first anatomical structure 71 and an end 22 of the second flexible elongated element 20 is constrained to a second anatomical structure 72 connected to the first structure by the damaged ligament or tendon that is intended to be replaced by the prosthesis. Once connected the end 12 and 22 to the respective anatomical structures, the elongated flexible elements 10 and 20 are arranged according to a configuration (figure 3) that allows a relative movement to it. More in detail, the desired relative location of the elongated flexible elements 10 and 20 is obtained causing the loop 21 to slide along the first elongated flexible element 10 up to a determined point, going beyond possible enlargements 11. Then, the loop 21 is made taut on the elongated flexible element 10 obtaining a first tightened configuration. During the surgical operation the length of the prosthesis is checked so that a correct relative movement between the two anatomical structures 71 and 72 is assured. If the length is not correct, the surgeon must simply enlarge the loop 21 and cause it to slide along the first element 10 up to a next or previous knot 11 in order to cause the prosthesis to achieve the correct length. The steadiness of the tightened configuration is ensured, in particular, by the tension on the loop 21 and by the presence of the knots 11 that prevent the tightened loop from a further sliding during operative conditions.

The above described prosthesis allows speeding up remarkably the surgical operations and to save material, since the ease with which it can be shifted from the positioning configuration to the tightened configuration assists the achievement of the correct length avoiding any replacement of the prosthesis.

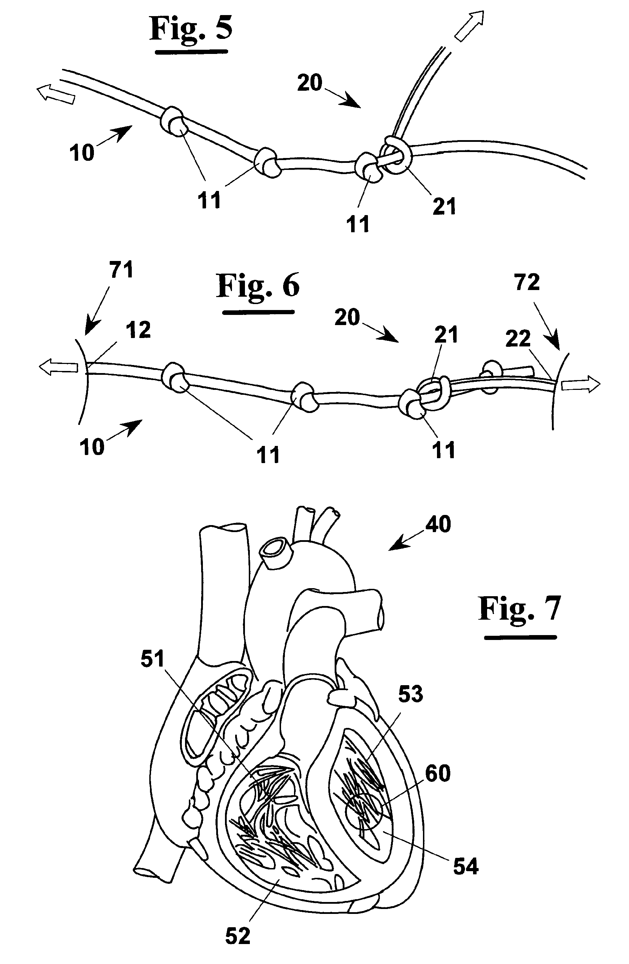

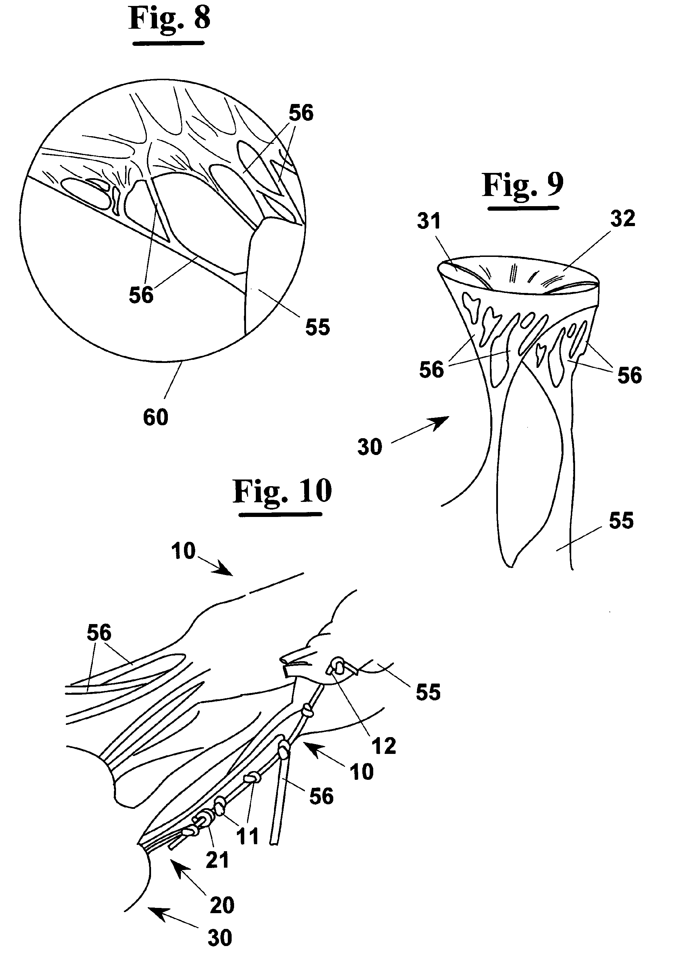

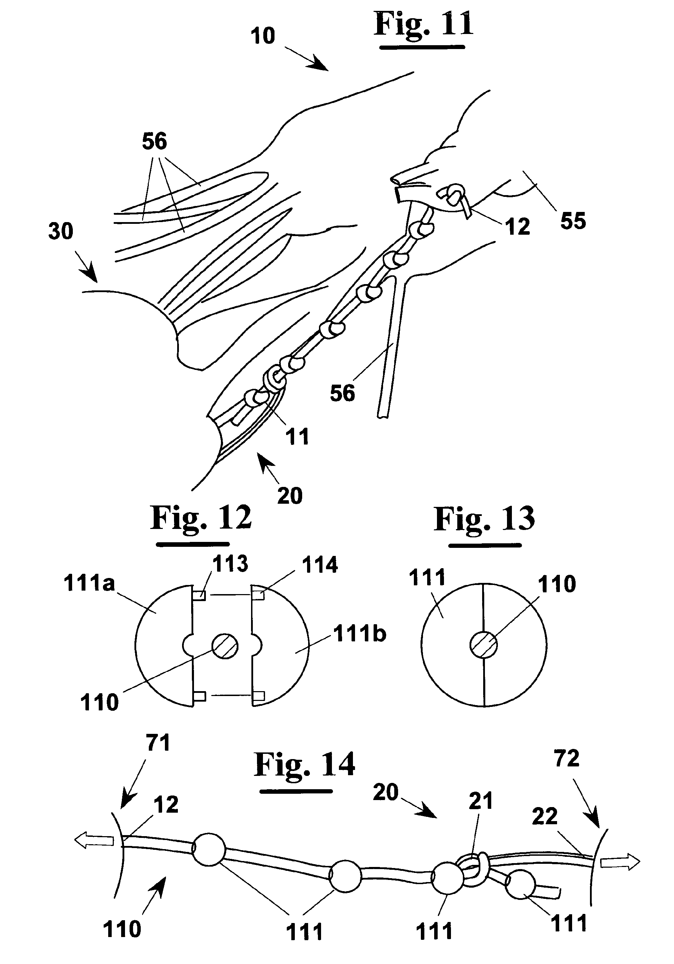

As described hereafter, with reference to figures 10 and 11, the prosthesis as above defined can be used for replacing damaged tendinous cords of a cardiac valve. As well known, the human heart 40 (figure 7) is divided in four cavities, two upper cavities, the right atrium 51 and the left atrium 53, and two lower cavities, the right ventricle 52 and the left ventricle 54. In particular, the left atrium 53 communicates with the left ventricle 54 that delivers blood to the remainder of the body. Atrio-ventricular flow is controlled by the mitral valve 30, diagrammatically shown in figure 9, which prevents the blood pushed by the atrium 53 towards the ventricle 54 from flowing back. The two leaflets 31 and 32 of the mitral valve 30 are held by the tendinous cords 56 fixed to the papillary muscles 55 that control their tension. Therefore, in the case shown in figures 10 and 11, if it is necessary to replace a tendinous cord with a prosthesis according to the invention, the end 12 of the first elongated flexible element 10 should be constrained to a papillary muscle 55 and the end 22 of the second flexible element 20 should be constrained to leaflet of the mitral valve 30. Once achieved a length of the prosthesis that allows the correct opening and closing of the mitral valve 30, restoring the function of the replaced tendinous cord, the loop 21 is turned into the tightened configuration preventing any movement from the actual position.

In figures 12 and 13 a possible exemplary embodiment is shown to make the enlargements 11 of figures above described. In this case, the enlargements 111 provide two halves 111a and 111b coupled by means of pins 113 that can engage in suitable housings 114. Each half 111a and 111b, has hemispheric shape, and provides a concave recess 116. When the reference element 111 is fixed to the elongated flexible element 110, the latter engages in concavity 116 that once coupled to the other forms a hole slightly smaller than the cross section of flexible element 110, in order to grip it preventing any relative movement.

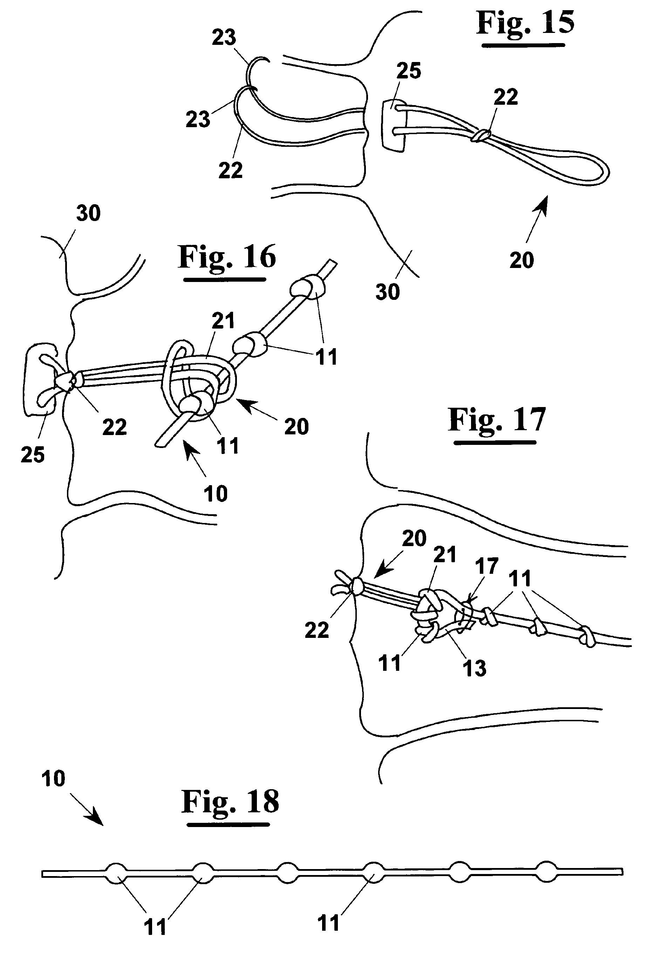

To assist the introduction of the elongated element 20 in the anatomical structure, for example in case of prosthesis of tendinous cords for a mitral valve leaflet 30 (figure 15), at least one metal needle 23 can be provided. Once put the elongated element 20 in the mitral valve leaflet 30 a fastening knot is made (not shown in the figure) and its end is drawn by the needles 23.

In the exemplary embodiment shown in figures 15 and 16 the elongated element 20 is, furthermore, associated to a contact plate 25, or pledget, which avoids damaging the native tissue of the structure.

In figure 17 the possibility is shown of fastening with a surgical suture 17 element 10 to element 20 at the loop 21, for giving further steadiness and avoiding any sliding on the knot.

Finally, in figure 18 an elevational side view is shown of a further exemplary embodiment of the elongated element 10 where the enlargements 11 are provided directly integrated in the material of which the prosthesis or the suture filament is made. This allows a much easier production, a constant distance between the enlargements 11, and avoids possible sliding on the enlargements 11.

The foregoing description of a specific embodiment will so fully reveal the invention according to a conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. A surgical device for connecting two anatomical structures, such as a prosthesis for tendons, ligaments and tendinous cords, or a pulling suture element, comprising:
- a first elongated flexible element suitable for being constrained at one end to a first anatomical structure;
- a second elongated flexible element suitable for being constrained at one end to a second anatomical structure, said second anatomical forming with the first anatomical structure an articular junction;
**characterised in that** said first and said second elongated flexible element have adjustable tightening means providing a mutual engagement to achieve a measured length of the device.

2. Surgical device, according to claim 1, wherein said adjustable tightening means moves from a positioning configuration that allows a relative movement of said flexible elongated elements to a tightened configuration in which said flexible elongated elements are integral to each other.

3. Surgical device, according to claim 1, wherein said adjustable tightening means provide:
- a slipknot or loop executed at said free end of said second elongated flexible element;
- a plurality of enlargements distributed at least on one portion of said first elongated flexible element, said enlargements being suitable for defining determined points at which said slipknot or loop can be fixed to provide a desired resulting length.

4. Surgical device, according to claim 1, wherein said enlargements are sized such that they block the run of said loop along said first elongated flexible element.

5. Surgical device, according to claim 1, wherein said enlargements are selected from the group comprised of:
- at least one knot;
- at least one element integrated in the body of the material of the flexible element;
- at least one element constrained to said first elongated flexible element in a stiff way;
- at least one element constrained to said first elongated flexible element in a way sliding longitudinally to it;
- a combination thereof.

6. Surgical device, according to claim 1, wherein said elongated flexible elements are constrained to said anatomical structures, at said end, by means of fastening means.

7. Surgical device, according to claim 1, wherein said each elongated flexible element has at one end at least one needle suitable to assist its introduction in the anatomical structure.

8. Surgical device, according to claim 1, wherein said elongated flexible elements are made of a material selected from the group comprised of:
- poliethylenterephtalate;
- politetrafluorethylene;
- polyaramid fibres;
- tetrapoliethylene.

9. Surgical device, according to the previous claims, **characterised in that** it is used as prosthesis for tendons, ligaments, tendinous cords.

10. Surgical device, according to claims from 1 to 8, **characterised in that** it is used as pulling suture element.
